# FASCICULE DE BREVET EUROPEEN

(11) **EP 3 261 803 B1**
(45) Date de publication et mention de la délivrance du brevet: **14.08.2019**
(21) Numéro de dépôt: 16714983.0
(22) Date de dépôt: 25.02.2016
(51) Int. Cl.: B21J 15/02, B21J 15/04, B25B 7/08, A61B 17/28, B26B 13/28, B26B 17/00, F16C 11/04

(54) **DISPOSITIF DE RIVETAGE POUR ASSEMBLAGE DE PRECISION**
NIETVORRICHTUNG FÜR PRÄZISIONSANORDNUNG
RIVETING DEVICE FOR PRECISION ASSEMBLY

(30) Priorité: 25.02.2015 FR 1551623
(43) Date de publication de la demande: 03.01.2018
(73) Titulaire: Gillet Group, 52800 Nogent (FR)
(72) Inventeur: PETIT, Fabrice, 52250 Orcevaux (FR); GILLET, Pascal, 52800 Nogent (FR)
(74) Mandataire: Oudin, Stéphane
(86) Numéro de dépôt international: PCT/FR2016/050440
(87) Numéro de publication internationale: WO 2016/135427

(56) Documents cités:
- EP-A1- 1 719 589
- CH-A- 271 455
- CN-U- 2 103 411
- DE-A1- 10 231 369
- DE-U1- 9 307 010
- FR-A- 1 101 870
- FR-A1- 2 715 594

## Description

### Domaine technique

La présente invention concerne de manière générale le domaine de l'outil à main tel que, par exemple, une pince coupante, une pince à bec ou encore une pince à dénuder. L'invention concerne plus particulièrement un dispositif de rivetage permettant d'assembler avec précision les deux branches dudit outil à main.

### Technique antérieure

Dans le domaine de l'outil à main, on connait déjà de nombreux modèles de pince comportant deux branches rigides articulées entre-elles autour d'un axe perpendiculaire au plan desdites branches et formant les manches de ladite pince, et un organe de travail aux extrémités des branches opposées de celles qui constituent lesdits manches.

Pour réaliser l'articulation des deux branches entre elles, il est connu d'utiliser une vis et un écrou de forme spécifique permettant la solidarisation des branches tout en gardant la possibilité de faire pivoter une branche par rapport à l'autre autour de l'axe longitudinal de la vis. Pour rendre les branches indémontables, la vis est collée dans l'écrou. Toutefois, ce type d'assemblage n'est pas satisfaisant car il nécessite la fabrication souvent onéreuse d'un ensemble vis - écrou spécifique et une dernière opération de collage difficilement industrialisable.

Pour pallier les inconvénients précédemment cités, il est également connu d'employer des dispositifs de rivetage tels que celui divulgué dans la demande de brevet FR 1 363 299. Ledit dispositif de rivetage est formé par deux pièces en matière élastiquement déformable et comporte, d'une part, un rivet interne à corps tubulaire et tête débordante et, d'autre part, un rivet externe à corps tubulaire et tête débordante comprenant un noyau s'étendant à l'intérieur dudit corps perpendiculairement à ladite tête, ledit noyau ayant un diamètre légèrement supérieur au diamètre interne du corps du rivet interne, mais étant de moindre longueur. Ledit corps tubulaire du rivet externe a un diamètre externe légèrement inférieur à celui des alésages des branches de pince à assembler et un diamètre interne au plus égal au diamètre externe du corps du rivet interne, et est de longueur au moins égale à celle de ce dernier et au plus égale à la somme des épaisseurs des branches à assembler. Avec cette configuration des moyens de rivetage, l'assemblage est alors obtenu en insérant à force, dans le corps du rivet externe, le corps du rivet interne qui va s'élargir sous l'effet du noyau et provoquer le serrage entre les rivets interne et externe, l'insertion va se poursuivre jusqu'à ce les têtes desdits rivets interne et externe viennent en contact avec les faces externes des branches à assembler.

Toutefois, ce dispositif de rivetage, qui est certes efficace, présente plusieurs inconvénients majeurs. Tout d'abord, le rivet externe est particulièrement complexe et donc onéreux à réaliser. Par ailleurs, le serrage s'effectue à l'intérieur des alésages des branches à assembler de sorte qu'il est impossible de le contrôler. Il n'est pas non plus possible de contrôler l'insertion du rivet interne dans le rivet externe et donc de garantir le bon fonctionnement de l'articulation entre les branches. Enfin, avec ce type de dispositif de rivetage, on ne peut pas garantir qu'il n'y aura pas de déplacement relatif des organes de travail disposés aux extrémités des branches opposées de celles qui constituent les manches. Ce dernier inconvénient est particulièrement problématique lorsque lesdits organes de travail sont des parties coupantes qui doivent impérativement être en vis-à-vis, car, s'il y a eu un déplacement relatif des organes de travail, il sera alors nécessaire de reprendre par limage lesdites parties coupantes, ce qui est une opération longue, coûteuse et interdisant tout usinage définitif des branches avant assemblage.

On connaît également un dispositif de rivetage tel que celui divulgué dans modèle d'utilité chinois CN 2 103 411. Toutefois, ce dispositif, qui permet d'éliminer l'écart généré entre deux lames en raison de l'usure en rapprochant lesdites lames l'une de l'autre, ne peut donc pas garantir un non-déplacement relatif des organes de travail disposés aux extrémités des branches opposées de celles qui constituent les manches.

### Exposé de l'invention

Le but de la présente invention est donc de pallier les inconvénients précédemment cités et de proposer un dispositif de rivetage permettant d'assembler avec précision entre elles deux branches articulées d'un outil à main, ledit dispositif garantissant un parfait fonctionnement de l'articulation et le non déplacement relatif des organes de travail disposés aux extrémités des branches opposées de celles qui constituent les manches. En outre, avec le dispositif de rivetage selon l'invention, les branches pourront être usinées de manière définitive avant leur assemblage permettant une industrialisation rationnelle du processus de fabrication de l'outil à main.

Conformément à l'invention, il est donc proposé un dispositif de rivetage pour assembler entre elles deux pièces comportant chacune un alésage et étant articulées autour de l'axe desdits alésages, ledit dispositif comportant un rivet externe comprenant un corps tubulaire de section transversale extérieure circulaire apte à être simultanément inséré dans l'alésage des deux pièces et muni à l'une de ses extrémités longitudinales d'une bride, et un rivet interne comprenant un corps cylindrique plein apte à être insérer à l'intérieur du corps tubulaire et issu sensiblement perpendiculairement d'un flasque, ledit corps tubulaire ayant un diamètre extérieur légèrement inférieur au diamètre desdits alésages et une longueur D entre la face arrière de sa bride et son extrémité opposée légèrement supérieure à la somme des épaisseurs des pièces, ledit corps cylindrique ayant une section transversale extérieure homothétique et de dimensions légèrement inférieures à la section transversale intérieure du corps tubulaire, ledit dispositif étant remarquable en ce que le rivet externe est indéformable et en ce que le corps cylindrique du rivet interne est déformable afin de sertir le rivet interne sur ledit rivet externe et a une longueur légèrement supérieure à celle du corps tubulaire lorsque que le dispositif de rivetage est mis en place sur les pièces à assembler, c'est-à-dire lorsque la face arrière de ladite bride est en contact avec l'une desdites pièces et la face arrière dudit flasque est en contact avec l'autre desdites pièces.

On comprend bien qu'avec une telle configuration, les risques liés au serrage des deux branches entre elles, à leur déformation et à leur déplacement relatifs sont de fait inexistants, puisque seul le rivet interne se déforme et que l'écartement entre les faces arrière des brides et flasque est garanti par le rivet externe indéformable. Il n'est donc plus nécessaire de reprendre l'usinage des organes de travail de l'outil à main après l'assemblage de ses branches entre elles.

Les sections transversales extérieure dudit corps cylindrique et intérieure du corps tubulaire sont avantageusement circulaires.

De manière préférée, le corps tubulaire comporte du côté de la bride un chanfrein aménagé le long de son bord circulaire intérieur.

Le rivet interne comporte de préférence un poinçonnage sur la face transversale d'extrémité de son corps cylindrique opposée au flasque

### Description sommaire des figures

D'autres avantages et caractéristiques ressortiront mieux de la description qui va suivre, d'un exemple particulier de réalisation, donné à titre d'exemple non limitatif, d'un dispositif de rivetage pour assembler les branches articulées d'un outil à main conforme à l'invention, en référence aux dessins annexés sur lesquels :
- la figure 1 est une vue de côté d'un outil à main dont les deux branches articulées sont assemblées par le dispositif de rivetage conforme à l'invention,
- la figure 2 est une section transversale agrandie de l'outil à main de la figure 1 selon l'axe II-II',
- la figure 3 est une vue agrandie d'un détail de la figure 2.

### Meilleure manière de réaliser l'invention technique

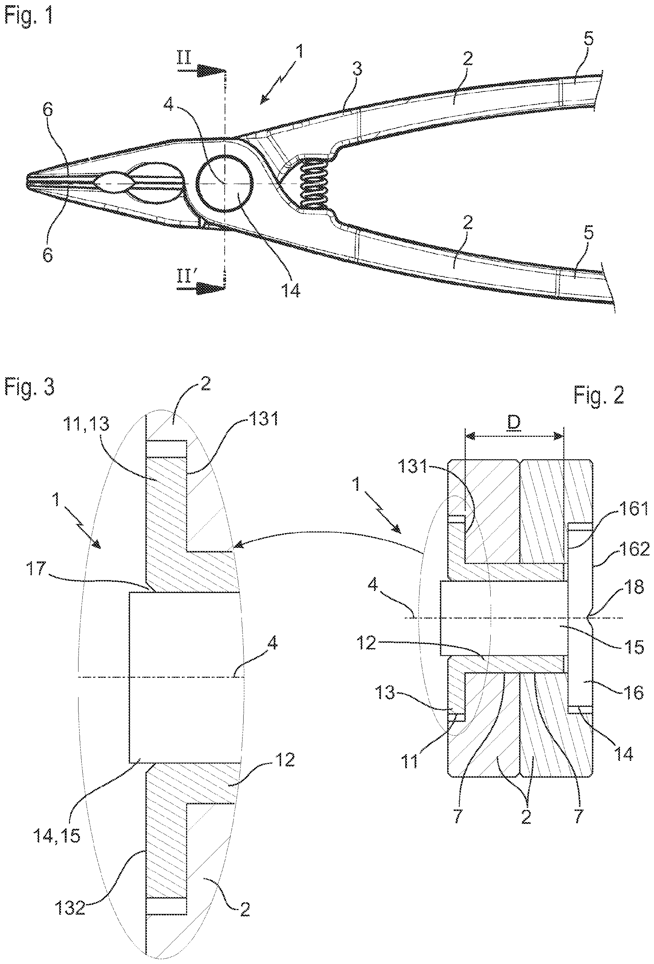

En référence aux figures 1 à 3, le dispositif de rivetage 1 permet l'assemblage entre deux pièces 2 articulées entre-elles autour d'un axe 4 perpendiculaire au plan desdites pièces 2, ces dernières étant par exemple deux branches 2 rigides d'un outil à main 3 formant les manches 5 dudit outil à main 3 et comportant chacune un organe de travail 6 à leur extrémité opposée de celle qui constituent le manche 5.

Ledit dispositif de rivetage 1 comporte un rivet externe 11 comprenant un corps tubulaire 12 de section transversale extérieure circulaire muni à l'une de ses extrémités longitudinales d'une bride 13 de forme globalement annulaire, et un rivet interne 14 comprenant un corps cylindrique 15 plein issu sensiblement perpendiculairement de la face arrière 161 d'un flasque 16 en forme générale de disque.

On désigne ici par "face arrière" la face du flasque 16 du rivet interne 14 ou de la bride 13 du rivet externe 11 qui est située du côté de la branche 2 associée lorsque l'outil à main 3 est assemblé à l'aide du dispositif de rivetage 1 conforme à l'invention, et par "face avant" la face opposée.

On comprend bien que la face avant 131 de la bride 13 du rivet externe 11 et/ou la face avant 161 du flasque 16 du rivet interne 14 sont avantageusement planes. Toutefois, elles pourront également être non planes, pour s'adapter aux pièces 2 à assembler, et être, par exemple, tronconiques, sans sortir du cadre de la présente invention.

Le diamètre extérieur du corps tubulaire 12 du rivet externe 11 est légèrement inférieur au diamètre des alésages 7 des branches 2 de l'outil à main 3 à assembler, de sorte à permettre le coulissement dudit rivet externe 11 à l'intérieur des alésages 7. En outre, la longueur D entre la face arrière 131 de sa bride 13 et l'extrémité opposée de son corps tubulaire 12 (Cf. figure 2) est au plus égale à la somme des épaisseurs des branches 2 à assembler.

De même, le corps cylindrique 15 du rivet interne 14 a, d'une part, un diamètre extérieur légèrement inférieur au diamètre intérieur du corps tubulaire 12 du rivet externe 11, de sorte à permettre le coulissement dudit rivet interne 14 à l'intérieur du rivet externe 11 et, d'autre part, une longueur légèrement supérieure à celle du corps tubulaire 12 du rivet externe 11 pour que, lorsque le dispositif de rivetage est mis en place sur les pièces 2 à assembler, le rivet interne 14 étant alors inséré dans le rivet externe 11 jusqu'à ce que les faces arrières 131 et 161 respectives de la bride 13 et du flasque 16 viennent en butée contre les pièces 2, son corps cylindrique 15 s'étende au-delà de l'autre extrémité dudit corps tubulaire 12 (Cf. figure 3).

Par ailleurs, le corps cylindrique 15 du rivet interne 14 est déformable et le rivet externe 11 est au contraire indéformable. On désigne ici par "indéformable" le fait que l'élément associé ne soit pas déformé lorsqu'il est soumis aux efforts mis en oeuvre pour assembler ledit dispositif de rivetage 1.

Pour ce faire, le rivet externe 11, qui est à l'instar du rivet interne 14 de préférence métallique, subit des traitements thermiques de sorte à éviter toute déformation sous l'action des efforts mis en oeuvre pour assembler entre eux lesdits rivets externe 11 et interne 14.

L'Homme du Métier n'aura aucune difficulté à choisir les métaux et traitements thermiques adaptés pour réaliser lesdits rivets externe 11 et interne 14 en fonction notamment de leurs dimensions et des efforts à mettre en oeuvre.

Par ailleurs, la section transversale intérieure du corps tubulaire 12 du rivet externe 11 et la section transversale extérieure du corps cylindrique 15 du rivet interne 14 pourront ne pas être circulaire sans sortir du cadre de la présente invention.

Avec cette configuration, pour assembler les deux branches 2 de l'outil à main 3, on procède de la manière suivante :
- on insère le rivet externe 11 simultanément dans l'alésage 7 de chacune des deux branches 2 de l'outil à main 3 jusqu'à ce que la face arrière 131 de sa bride 13 vienne en contact avec l'une desdites branches 2, ces dernières étant positionnées entre elles conformément à la figure 1,
- on insère ensuite le rivet interne 14 dans le corps tubulaire 12 du rivet externe 11 jusqu'à ce que la face arrière 161 de son flasque 16 vienne en contact avec l'autre desdites branches 2, l'extrémité libre du corps cylindrique 15 du rivet interne 14 dépassant alors légèrement de la bride 13 du rivet externe 11,
- on exerce enfin une pression, par le biais de l'outil d'une presse, sur l'extrémité libre du corps cylindrique 15 du rivet externe 11, dont l'autre extrémité côté flasque 16 est en butée sur le bâti de la presse, pour la déformer de sorte à créer un bourrelet afin de sertir le rivet interne 14 sur le rivet externe 11 et réaliser ainsi l'assemblage entre elles des deux branches 2 de l'outil à main 3.

On comprend bien qu'avec le dispositif de rivetage 1 selon l'invention, seule l'extrémité libre du corps cylindrique 15 se déforme, ce qui permet de contrôler l'opération de déformation et de garantir le bon fonctionnement de l'articulation desdites branches 2 car le rivet externe 11 est indéformable sous l'action des efforts mis en oeuvre et le diamètre extérieur de son corps tubulaire 12 demeure invariable et garantit le jeu nécessaire au bon fonctionnement de l'articulation desdites branches 2.

Par ailleurs, les pièces 2 ne sont pas serrées entre elles car seul le corps cylindrique 15 est déformable et que la quantité de matière à déformer, pour assurer le sertissage entre les rivets externe 11 et interne 14, est minime, à savoir l'extrémité libre du corps cylindrique 15 du rivet interne 14 dépassant alors légèrement du rivet externe 11. Il faut donc que la presse soit réglée de sorte que sa force de compression soit juste suffisante pour que l'outil ne puisse déformer que l'extrémité libre du corps cylindrique 15 et que la progression dudit outil s'arrête lorsqu'il arrive en contact avec la bride 13 du rivet externe 11. En effet, dans la mesure où le rivet externe 11 est indéformable et donc plus résistant que ledit corps cylindrique 15, il serait nécessaire de mettre en oeuvre une force de compression nettement plus élevée que celle nécessaire à la déformation de l'extrémité libre du corps cylindrique 15, ce qui n'est pas possible compte tenu du réglage de la presse.

Avec cette configuration du dispositif de rivetage 1, on peut obtenir un assemblage très précis des deux branches 2 puisque ledit diamètre extérieur du corps tubulaire 12 du rivet externe 11 peut être réalisé avec une précision de l'ordre du 1/100^{ème} de millimètre.

Ainsi, les risques liés au serrage des deux branches 2 entre elles, à leur déformation et à leur déplacement relatifs sont de fait inexistants, puisque les efforts ne sont exercés que sur l'extrémité libre du corps cylindrique 15 du rivet interne 14 et que l'écartement entre les faces arrière des brides 13 et flasque 16 est garanti par l'indéformabilité du rivet externe 11.

Le dispositif de rivetage 1 permet d'éviter toute reprise des organes de travail 6 de l'outil à main 3 après l'assemblage de ses branches 2 entre elles, et donc de pouvoir industrialiser la fabrication de l'outil à main 3 en assemblant ses branches 2 à l'issue de leur usinage définitif.

De même, le rivet externe 11 indéformable évite toute déformation intempestive du rivet interne 14, c'est-à-dire autre que celle de l'extrémité libre de son corps cylindrique 15.

Pour faciliter la déformation de ladite extrémité libre du corps cylindrique 15, le corps tubulaire 12 comporte du côté de la bride 13 un chanfrein 17 aménagé le long de son bord circulaire intérieur.

Enfin, la face avant 162 du flasque 16 du rivet interne 14 comporte de préférence un poinçonnage 18 permettant de positionner correctement le dispositif de rivetage 1 sous l'outil de la presse lors du sertissage dudit rivet interne 14 sur le rivet externe 11.

### Possibilité d'application industrielle

On comprend bien que le dispositif de rivetage 1 selon l'invention peut être utilisé pour tout type d'outil autre qu'un outil à main 3 du type pince ou analogue. De même, il est bien évident que le dispositif de rivetage 1 conforme à l'invention peut être adapté pour assembler deux pièces articulées entre elles ou non et appartenant à un tout autre type de mécanisme technique tel que, par exemple, un moteur ou encore un élévateur.

Enfin, il va de soi que les exemples de dispositif de rivetage 1 conformes à l'invention qui viennent d'être décrits ne sont que des illustrations particulières, en aucun cas limitatives de l'invention.

## Revendications

1. Dispositif (1) de rivetage pour assembler entre elles deux pièces (2) comportant chacune un alésage (7) et étant articulées autour de l'axe (4) desdits alésages (7), ledit dispositif (1) comportant un rivet externe (11) comprenant un corps tubulaire (12) de section transversale extérieure circulaire apte à être simultanément inséré dans l'alésage (4) des deux pièces (2) et muni à l'une de ses extrémités longitudinales d'une bride (13), et un rivet interne (14) comprenant un corps cylindrique (15) plein apte à être insérer à l'intérieur du corps tubulaire (12) et issu sensiblement perpendiculairement d'un flasque (16), ledit corps tubulaire (12) ayant un diamètre extérieur légèrement inférieur au diamètre desdits alésages (7) et une longueur D entre la face arrière (131) de sa bride (13) et son extrémité opposée au plus égale à la somme des épaisseurs des pièces (2), ledit corps cylindrique (15) ayant une section transversale extérieure homothétique et de dimensions légèrement inférieures à la section transversale intérieure du corps tubulaire (12), ledit dispositif (1) étant **caractérisé en ce que** le rivet externe (11) est indéformable et **en ce que** le corps cylindrique (15) du rivet interne (14) est déformable afin de sertir le rivet interne (14) sur ledit rivet externe (11) et a une longueur légèrement supérieure à celle du corps tubulaire (12) lorsque que le dispositif de rivetage (1) est mis en place sur les pièces (2) à assembler, c'est-à-dire lorsque la face arrière (131) de ladite bride (13) est en contact avec l'une desdites pièces (2) et la face arrière (161) dudit flasque (16) est en contact avec l'autre desdites pièces (2).

2. Dispositif (2) de rivetage selon la revendication 1 **caractérisé en ce que** les sections transversales extérieure dudit corps cylindrique (15) et intérieure du corps tubulaire (12) sont circulaires.

3. Dispositif (2) de rivetage selon l'une quelconque des revendications 1 ou 2, **caractérisé en ce que** le corps tubulaire (12) comporte du côté de la bride (13) un chanfrein (17) aménagé le long de son bord circulaire intérieur.

4. Dispositif (2) de rivetage selon l'une quelconque des revendications 1 à 3, **caractérisé en ce que** le rivet interne (14) comporte un poinçonnage (18) sur la face avant (162) du flasque (16).

## Patentansprüche

1. Nietvorrichtung (1) zum Montieren miteinander von zwei Teilen (2), die jeweils eine Bohrung (7) beinhalten und um die Achse (4) der Bohrungen (7) angelenkt sind, wobei die Vorrichtung (1) einen äußeren Niet (11) beinhaltet, der einen röhrenförmigen Körper (12) mit kreisförmigem Außenquerschnitt umfasst, der imstande ist, zugleich in die Bohrung (4) der beiden Teile (2) eingeführt zu werden, und an einem seiner Längsenden mit einem Flansch (13) ausgestattet ist, und einen inneren Niet (14), der einen vollen zylindrischen Körper (15) umfasst, der imstande ist, ins Innere des röhrenförmigen Körpers (12) eingeführt zu werden, und im Wesentlichen senkrecht aus einem Lagerschild (16) kommend, wobei der röhrenförmige Körper (12) einen Außendurchmesser aufweist, der etwas kleiner ist als der Durchmesser der Bohrungen (7) und eine Länge D zwischen der Rückseite (131) seines Flansches (13) und seinem entgegengesetzten Ende höchstens gleich der Summe der Stärken der Teile (2), wobei der zylindrische Körper (15) einen homothetischen Außenquerschnitt und mit Abmessungen aufweist, die etwas kleiner sind als der Innenquerschnitt des röhrenförmigen Körpers (12), wobei die Vorrichtung (1) **dadurch gekennzeichnet ist, dass** der äußere Niet (11) nicht verformbar ist, und dadurch, dass der zylindrische Körper (15) des inneren Niets (14) verformbar ist, um den inneren Niet (14) auf dem äußeren Niet (11) zu crimpen, und eine etwas größere Länge als jene des röhrenförmigen Körpers (12) aufweist, wenn die Nietvorrichtung (1) auf den zu montierenden Teilen (2) angebracht wird, das heißt, wenn die Rückseite (131) des Flansches (13) in Kontakt mit dem einen der Teile (2) ist und die Rückseite (161) des Lagerschildes (16) in Kontakt mit dem anderen der Teile (2) ist.

2. Nietvorrichtung (2) nach Anspruch 1, **dadurch gekennzeichnet, dass** der Außenquerschnitt des zylindrischen Körpers (15) und der Innenquerschnitt des röhrenförmigen Körpers (12) kreisförmig sind.

3. Nietvorrichtung (2) nach einem der Ansprüche 1 oder 2, **dadurch gekennzeichnet, dass** der röhrenförmige Körper (12) auf Seiten des Flansches (13) eine Fase (17) beinhaltet, die entlang seines inneren kreisförmigen Randes angeordnet ist.

4. Nietvorrichtung (2) nach einem der Ansprüche 1 bis 3, **dadurch gekennzeichnet, dass** der innere Niet (14) eine Lochung (18) auf der Vorderseite (162) des Lagerschildes (16) beinhaltet.

## Claims

1. Riveting device (1) for assembling two parts (2) together, each comprising a bore (7) and being articulated about the axis (4) of said bores (7), said device (1) comprising an outer rivet (11) comprising a tubular body (12) of circular outer transversal cross-section, capable of being simultaneously inserted into the bore (4) of the two parts (2) and provided with one of the longitudinal ends thereof of a flange (13), and an inner rivet (14) comprising a solid cylindrical body (15), capable of being inserted inside the tubular body (12) and coming substantially perpendicularly from a flange (16), said tubular body (12) having an outer diameter slightly less than the diameter of said bores (7) and a length D between the rear face (131) of the flange (13) thereof and the opposite end thereof at most equal to the sum of the thicknesses of the parts (2), said cylindrical body (15) having a homothetic outer transversal cross-section and of dimensions slightly less than the inner transversal cross-section of the tubular body (12), said device (1) being **characterised in that** the outer rivet (11) cannot be deformed and **in that** the cylindrical body (15) of the inner rivet (14) can be deformed in order to crimp the inner rivet (14) on said outer rivet (11) and has a length slightly greater than that of the tubular body (12) when the riveting device (1) is implemented on the parts (2) to be assembled, i.e. when the rear face (131) of said flange (13) is in contact with one of said parts (2) and the rear face (161) of said flange (16) is in contact with the other of said parts (2).

2. Riveting device (2) according to claim 1, **characterised in that** the outer transversal cross-section of said cylindrical body (15) and the inner one of the tubular body (12) are circular.

3. Riveting device (2) according to any one of claims 1 or 2, **characterised in that** the tubular body (12) comprises, on the side of the flange (13), a chamfer (17) arranged along the inner circular edge thereof.

4. Riveting device (2) according to any one of claims 1 to 3, **characterised in that** the inner rivet (14) comprises a punch (18) on the front face (162) of the flange (16).
